# EUROPEAN PATENT APPLICATION

(11) **EP 1 003 032 A1**
(43) Date of publication of application: **24.05.2000**
(21) Application number: 98870254.4
(22) Date of filing: 17.11.1998
(51) Int. Cl.: G01N 27/327, C12Q 1/00, G01N 33/543

(54) **Sensor comprising an oligomer binding layer and method of making such sensor and arrays of such sensors**

(71) Applicant: INTERUNIVERSITAIR MICRO-ELEKTRONICA CENTRUM VZW, 3001 Heverlee (BE); UNIVERSITAIRE INSTELLING ANTWERPEN, B-2610 Wilrijk (BE)
(72) Inventor: Huyberechts, Guido, 1820 Melsbroek (BE); Jordens, Sven, 3540 Herk-de-Stad (BE)
(74) Representative: Van Malderen, Joelle

(57) **Abstract**

An aim of the invention is to provide a new type of sensor, capable of recognising and/or quantifying analytes in a fluid. A further aim of the present invention is to provide such sensors with an oligomer material as a binding layer. A further aim of the present invention is to provide a novel method for the manufacture if such sensor wherein the oligomer layer is locally deposited on the sites of the sensor having a multitude of sensing sites.

## Description

### Field of the invention

The present invention relates to sensors and to methods for the making thereof.

### State of the art

Sensors and biosensors are known in the art. A biosensor can be defined as a system that can determine the existence or the concentration of a certain analyte in a sample by translating molecular recognition of said analyte ultimately into an electrical signal by means of a translation system. Biosensors can be used for any kind of analyte that can be detected by biological means. Most important is the use for detecting and/or quantifying for instance metabolites, drugs, proteins, antigen-antibody interactions, i.e. metabolite detection and affinity and immunosensing in general. For instance, detecting glucose in a diabetes patient's blood is of vital importance, detection of possibly life-threatening micro-organisms in food enhances food safety, detection and quantification of pollutants like CO, herbicides, chemicals and heavy metals are necessary to find and decontaminate polluted areas.

Biosensors can be catalogued in different groups, depending on their biological recognition system and the translation system.

Subdivision according to the biological recognition system comprises enzymatic sensors (based upon the reaction of a substrate catalysed by an enzyme, immunosensors (based upon the affinity between antibodies (or parts thereof) and antigenic determinants, e.g. ELISA test) and genosensors (based upon recognition of complementary RNA and/or DNA single strand molecules, e.g. PCR).

Subdivision of the biosensors using a classification according to the translation system used, comprises electrochemical biosensors (amperometric, potentiometric, capacitive or impedimetric), optical biosensors (e.g. Surface Plasmon Resonance (SPR), ellipsometric, fluorescence, ...), gravimetric biosensors (measuring a difference in mass by measuring a change in resonance frequency of a quartz crystal when the analyte binds or adsorbs to the crystals), and calorimetric biosensors (measuring the reaction enthalpy released when the analyte binds to a substrate).

When using recognition biomolecules, such as antibodies, enzymes, oligonucleotides or nucleic acids, these molecules need to be fixed to a carrier surface in order to be able to perform their recognising function in a reproducible way. Several possible techniques have been devised to perform the immobilisation.

One technique that is used in glucose-sensors is the immobilisation of e.g. an enzyme (glucose-oxidase) between two selectively permeable membranes. The first membrane is only permeable to the analyte (glucose), while the second membrane is only permeable to hydrogen peroxide, a reaction product of the enzyme-promoted reaction of glucose. The amount of hydrogen peroxide can easily be measured by oxidation at an electrode and correlated to the amount of analyte in the measured sample.

Another technique relies on physical adsorption to a fixed carrier surface. This has the major advantage that no special agents are necessary to bind the biomolecules to the carrier surface, the adhesion being the result of weak interactions such as Van der Waals interactions, dipole-dipole interactions and/or hydrogen bonds.

A third technique is based upon bifunctional reagents that can couple molecules to each other. Such a configuration can attenuate a highly active protein layer by partly shielding the activity with inert protein material. A reagent frequently used in this context is glutaraldehyde. This reagent can react with two free amino groups.

Another technique is covalent binding to a substrate. Usually, in the case of binding a protein, this implies the binding of a reagent to a free amino group hat is unnecessary for the subsequent recognition of the analyte molecule. A regularly used way to perform this is to use a shielding molecule that reversibly binds to the active site of the biomolecule necessary for the analyte recognition while covalently fixing said biomolecule to the carrier substrate. Removal of the shielding molecule results in an active biosensor.

In the prior art, polymers are used to immobilise biomolecules that can recognise an analyte. The polymers are bound to an electrode surface of a sensor. Polymers can provide the necessary sites to induce adsorption by weak forces such as Van der Waals forces, dipole-dipole interactions and hydrogen bonds. Further, polymers can provide a matrix in which said biomolecules can be trapped. Polymers that are useful for this purpose are amongst many others PVA (poly(vinylalcohol)), PVC (poly(vinylchloride)), PAA (poly(acrylamide)) and PU (polyurethane). This solution provides only a slow response time, since analytes have to diffuse through the matrix to the biomolecules to be detectable.

The polymers can be deposited on one of the surfaces of the sensor by spin-coating. This method does not allow for a localized, selective deposition of the polymers. Dispensing of the polymers on localized spots of the sensor can be done but in this case the biomolecules are to be binded to the electrode prior to definition of the detection system (the sensor). Heat treatments however can destruct the biomolecules and/or the binding of the biomolecules to the polymers. The selective dispensing of the polymers can not be executed in strongly miniaturized (microsystem) sensors and the final configuration of the sensor system can not be tailored. The use of photolithography techniques in binding polymers to a sensor system is limited to transparent systems.

### Aims of the invention

An aim of the invention is to provide a new type of sensor, capable of recognising and/or quantifying analytes in a fluid. A further aim of the present invention is to provide such sensors with an oligomer material as a binding layer. A further aim of the present invention is to provide a novel method for the manufacture if such sensor wherein the oligomer layer is locally deposited on the sites of the sensor having a multitude of sensing sites.

### Summary of the invention

A first aspect of the present invention is to disclose a new type of oligomer material. These new oligomer materials can comprise substituted bis(styryl)benzene oligomer materials and substituted bis(styryl)thiopheen oligomer materials. In the sequel of this patent application, the bis(styryl)benzene oligomer materials and the bis(styryl)thiophene oligomer materials will be denoted as backbone oligomer materials. The substituents can comprise alkoxy-groups, hydroxyl-groups, amino-groups, epoxy-groups, thiol-groups and carboxyl-groups or any other group that is capable of binding recognition molecules for detecting analytes in a fluid. These groups can be a introduced by a spacer-arm on the oligomer backbone material. The spacer-arm can be an alkane-chain or a Si-chain or a alkene-chain or derivatives.

The backbone oligomer with an alkoxy-groups can be one of the group consisting of 2,5-dimethoxy-1,4-bis(3,4,5-trimethoxystyryl)benzene, 2,5-alkoxy-1,4-bis(3,4,5-trimethoxystyryl)benzene and 1,4-bis(3,4,5-trimethoxystyryl)thiophene.

It is a second aspect of the present invention to disclose a sensor for the detection of an analyte in a fluid, characterised in that said sensor comprises:
- a substantially inert carrier substrate ;
- at least one electrode positioned on at least part of said substrate ;
- an oligomer binding layer at least partly positioned on at least part of said electrode ; and
- a recognition molecule binded to said oligomer binding layer, said biomolecule being capable of recognising said analyte.

In a preferred embodiment of the invention, the oligomer binding layer is selected from the group of substituted bis(styryl)benzene oligomer materials and substituted bis(styryl)thiopheen oligomer materials. The substituents on these backbone oligomer materials can comprise alkoxy-groups, hydroxy-groups, amino-groups, epoxy-groups, thiol-groups and carboxyl-groups or any other group that is capable of binding molecules for detecting analytes in a fluid. The oligomer layer can also be a substituted arylene alkenylene oligomer. The chemical structure of arylene alkenylene oligomers can be defined as follows: every possible sequence of one or more aromatic or heteroaromatic units like benzene, thiophene, pyrrole, aniline, indole, and many others (the arylene segments) which can be connected by one or more alkenyl segments (-(CR=CR'-)n). The arylene alkenylene oligomer can be substituted as well on the aryl segments as on the alkenyl segments.

In a preferred embodiment of the invention, the sensor is a biosensor, preferably a impedimetric biosensor which has two electrodes. The binding of the recognition molecule to the oligomer binding layer can be a covalent bonding. Said recognition molecule can be selected from the group consisting of DNA, RNA, oligonucleotide, protein, antibody, antigen and/or enzyme molecule.

The invention further concerns a sensor array comprising at least two sensors as described hereabove. Such sensor array has a multitude of sensing sites, each sensing site being a sensor. Said sites can each have a different recognition molecule and/or oligomer binding layer. The sites can also have a different number or density of recognition molecules. A number of sites can have the same recognition molecule and/or density of recognition molecule and/or have the same oligomer binding layer.

Also, the invention concerns in a third aspect the use of oligomers materials for the manufacture of biosensors.

A method for the manufacture of a sensor is disclosed, said sensor having a substantially inert carrier substrate and at least one electrode positioned on at least part of said substrate, said method comprising the following steps:
- depositing an oligomer binding layer on said electrode, and
- binding a recognition molecule capable of recognition of an analyte in a fluid to said oligomer layer.

The recognition molecule can be binded to the oligomer layer prior to the deposition step or after the step of depositing the oligomer binding layer on the electrode.

Further is disclosed a method for locally depositing an oligomer binding layer on a sensor array comprising at least two sensors. The sensors have a substantially inert carrier substrate and at least one electrode positioned on at least part of said substrate. Such sensor array has a multitude of sensing sites, each sensing site being a sensor. In a preferred embodiment, the inert carrier substrate is a common substrate of the array. Said oligomer binding layer is deposited on at least one selected electrode of said array by applying a voltage higher than the threshold voltage for electrodeposition of said oligomer on said selected electrode while the voltage applied on at least part of the other electrodes of said array is lower than said threshold voltage. In a preferred embodiment of an impedimetric biosensor array which has two electrodes for one sensing site, the oligomer binding layer is deposited to both electrodes during this local deposition. The oligomer binding layer can be absent in-between the separate electrodes or the oligomer layer can cover the inert substrate in-between the electrodes.

### Brief description of the figures

- Figure 1: is representing an embodiment of an impedimetric biosensor with two electrodes which are configured in an interdigated, separate electrode structure.
- Figure 2: is representing the impedimetric biosensor of figure 1 with the detection principle.
- Figure 3: is representing a backbone oligomer : 2,5-dimethoxy-1,4-bis(3,4,5-trimethoxystyryl)benzene.
- Figure 4: is representing the synthesis pathway of the oligomer as described in Figure 3.
- Figure 5: is describing several possibilities of functional groups which are used for the coupling of a free amino group.
- Figure 6: is representing a backbone oligomer 2,5-alkoxy-1,4-bis (3,4,5-trimethoxystyryl)benzene.
- Figure 7: is representing a backbone oligomer : 1,4-bis (3,4,5-trimethoxystyryl)thiophene.
- Figure 8: is representing a sensor array having a 4x4 impedimetric sensor structure. According to this embodiment only line by line a voltage can be put on the sensing sites of the array. The local deposition technique of the invention in this embodiment therefore can be done only line by line.
- Figure 9: is representing a flow-injection analyser using a sensor array according to the invention.

### Detailed description of several embodiments of the present invention

The invention will be demonstrated using an impedimetric biosensor, as described in Van Gerwen et al., Nanoscaled interdigitated electrode arrays for biochemical sensors, Sensors and Actuators B 49 (1998) 73-80. The teaching of this document is incorporated herein by reference. This kind of sensor construction is used in all examples detailed herebelow.

The present invention can also be applied to a biosensor as described in WO97/21094, incorporated herein by reference.

These impedimetric biosensors have two electrodes. Sensors with only one electrode can also be used according to the invention. Such one electrode sensors can be gravimetric sensors or surface plasmon resonance sensors and can be fabricated. The electrode on the surface plasmon resonance sensors can be made by coating a film of gold on a prism and the oligomer layer is thereafter deposited on the gold film.

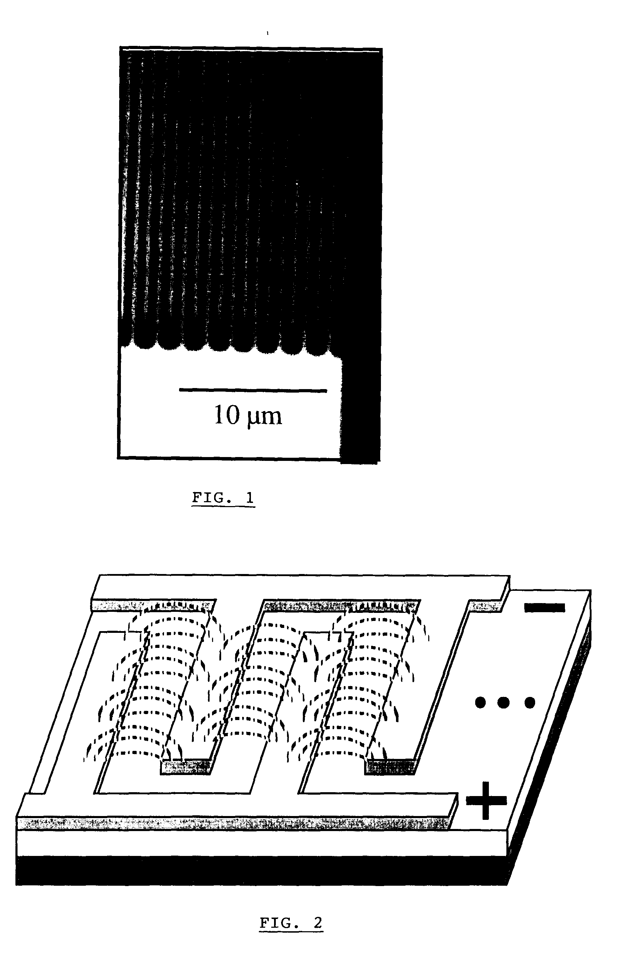

The nanoscaled interdigitated electrode arrays of Van Gerwen et al. can be realised by using deep UV lithography. Electrodes have a width of between 250 and 500 nm, with siliciumdioxide in between. The electrode material can comprise palladium, gold, platinum or titanium dioxide or any other electrode material that allows for electrodeposition of oligomer materials. In the case of palladium, a 50 nm layer can be deposited on a 15 nm titanium interface layer, deposited on an isolating, thermally grown siliconoxide layer. A Scanning Electronic Microscope (SEM) image is provided in Fig. 1. Preferably, and as indicated in this Fig. 1, the pattern used has a "interdigitated" layout.

The layout of the electrodes can be imposed by photolithography, followed by a lift-off procedure. An impedimetric signal can be detected when biomolecules attach to the structure and change the impedance spectrum. This impedance spectrum is measured by applying a sinusoidal alternating voltage to the electrode structure as previously described. The resulting currents are detected with changed amplitude and phase. The current and voltage are at all times related to each other by a complex impedance. This impedance can thus be calculated at all times, and an impedance spectrum, relating the measured impedance to the applied voltage frequency. This impedance spectrum can be represented by a Bode plot, where the two components |Z| (amplitude) and ϕ (orientation) are represented in function of the voltage frequency.

Such an impedance spectrum is then mimicked by building an equivalent electronic configuration of electrical and electrochemical components. From this electronic configuration scheme, the parameter that is influenced by the binding of the analyte with the probe attached to the electrode surface, can be identified.

An embodiment of the present invention comprises the use of oligomers for binding recognition molecules to such sensors. The molecules can covalently attach to said oligomers with functional groups that are not necessary to perform the recognition function necessary to detect an analyte. Two modi operandi are possible: the recognition molecules are binded or incorporated onto the oligomers prior to the deposition step (i.e. the deposition step deposits an oligomer-biorecognition ensemble), or the biomolecules are attached to a free functional group of the oligomer after the deposition step. Thus the deposition of the oligomer can create a localised sensor element activated for binding the recognition molecule).

The use of oligomers as hereafter described has the further advantage of allowing for local deposition on the sensor. This can be exploited to provide a sensor array, with which different analytes can be measured.

### Example 1

### a) Synthesis of a potential backbone oligomer 2,5-dimethoxy-1,4-bis(3,4,5-trimethoxystyryl)benzene

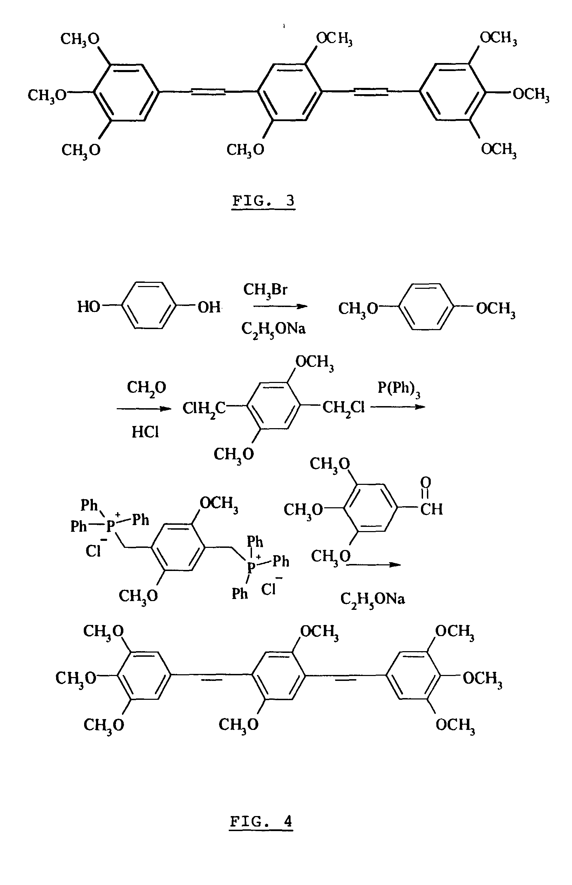

### (OMT:octamethoxytrimer) (Fig. 3)

OMT can be synthesised by the Wittig route, which implies the reaction of a phosphoniumylide with a carbonylfunction to result in a double bond. The synthesis pathway is depicted in Fig. 4. Hydroquinone is reacted with methylbromide to form 1,4-dimethoxybenzene (Williamson synthesis). The 2,5-dimethoxy-1,4-dichlorobenzene results from the chloromethylation of 1,4-dimethoxybenzene. Reaction with triphenylphosphine gives rise to the according phosphonium salt. In the presence of sodiumethanolate, phosphoniumylide is formed that can further react with 3,4,5-trimethoxybenzaldehyde.

### b) Electrodeposition of the oligomer

The oligomer can be electrodeposited by applying at least a certain threshold voltage on the electrode that has to be covered. This voltage can provide the energy to deposit the oligomer on the electrode.

### c) Coupling of the biomolecule to the oligomer

Coupling of the biomolecule (that will be responsible for the recognition of the analyte) to the oligomer can be performed by using one of the available functional groups. These groups can be substituted into the oligomer by known chemical methods and are preferably a hydroxyl, amino or carboxyl group. Said groups may be substituted on the arylrings and/or on the olefine bonds.

As an example, an amino group may be included in the 3-position of the central arylring of the exemplified compounds. Furthermore, a functional group may be incorporated which is bonded to the oligomer via a spacer arm, preferably an alkane chain, which facilitates the bonding of a biomolecule. After incorporation of this functional group, this oligomers may be electrochemically deposited and thereafter the recognition biomolecule can be bonded.

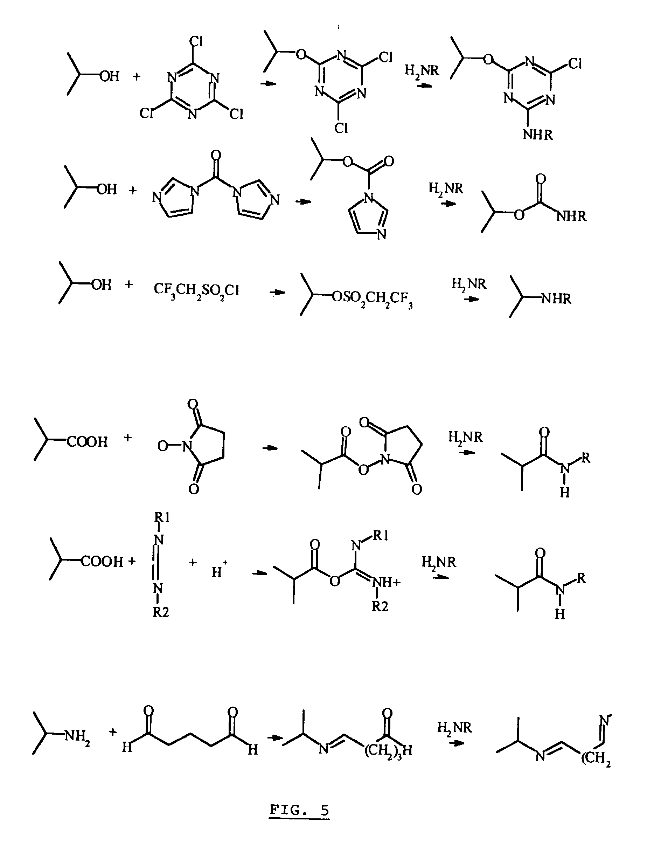

The coupling of a free amino group (as in proteins and peptides) to a functional group of one of the types listed higher can be seen in Fig. 5.

### d) Recording of base impedance spectrum, mimicking said impedance spectrum by an equivalent electronic configuration and calibrating the biosensor for an analyte

### Example 2

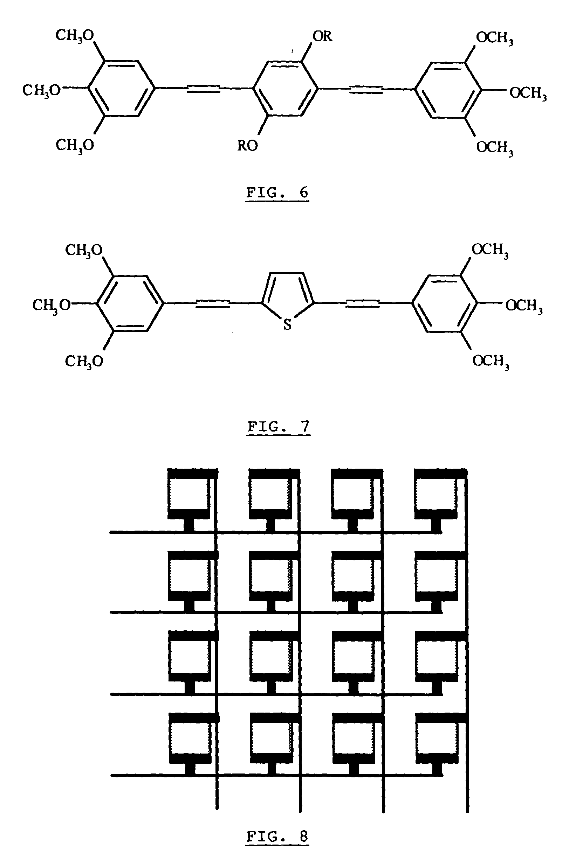

Same as example 1, but with 2,5-alkoxy-1,4-bis(3,4,5-trimethoxystyryl)benzene (Fig. 6) as backbone oligomer.

### Example 3

Same as example 1, but with 1,4-bis(3,4,5-trimethoxystyryl)thiophene (Fig. 7) as backbone oligomer.

### Example 4

Same as example 1, but with step c (attachment of the biomolecule to the oligomer) performed before step b (electrodeposition of the oligomer).

### Example 5

Same as example 2, but with step c (attachment of the biomolecule to the oligomer) performed before step b (electrodeposition of the oligomer).

### Example 6

Same as example 3, but with step c (attachment of the biomolecule to the oligomer) performed before step b (electrodeposition of the oligomer).

### Example 7

Sensor array with localised deposition (immobilisation).

A sensor array (e.g. 4x4 impedimetric sensor structure such as in Fig. 8) can be selectively coated with different oligomers and/or different biomolecules or different amounts of biomolecules (to enhance the range of the sensor array for one specific analyte). This can be done by selecting one electrode (line according to figure 8) of the array, electrodeposit only on this electrode by only applying a sufficient voltage on this electrode. Specific choices of biomolecules and/or oligomers depend on the application.

### Example 8

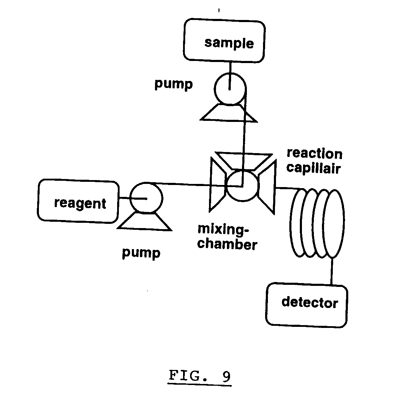

A sensor or sensor array according to the present invention can be used as a detector in a flow-injection analyser. Such a flow-injection analyser can be seen in Fig. 9.

## Claims

1. A sensor for the detection of an analyte in a fluid, said sensor comprising :
- a substantially inert carrier substrate ;
- at least one electrode positioned on at least part of said substrate ;
- an oligomer binding layer at least partly positioned on at least part of said electrode ; and
- a recognition molecule binded to said oligomer binding layer, said biomolecule being capable of recognising said analyte.

2. The sensor as recited in claim 1 wherein the oligomer binding layer is a substituted backbone oligomer material.

3. The sensor as recited in claim 2 wherein the substituents on the backbone oligomer materials comprise at least one of the group of alkoxy-groups, hydroxy-groups, amino-groups, epoxy-groups, thiol-groups and carboxyl-groups or any other group that is capable of binding recognition molecules for detecting analytes in a fluid.

4. The sensor as recited in claim 1 wherein the oligomer binding layer is a substituted arylene alkenylene oligomer.

5. Sensor as in any one of the preceding claims, wherein the oligomer layer is selected from the group consisting of 2,5-dimethoxy-1,4-bis(3,4,5-trimethoxystyryl) benzene, 2,5-alkoxy-1,4-bis(3,4,5-trimethoxystyryl)benzene and/or 1,4-bis(3,4,5-trimethoxystyryl) thiofene.

6. Sensor as recited in claim 1, wherein the binding of the analyte to the oligomer layer is a covalent bonding.

7. Sensor as in any one of the preceding claims, wherein said recognition molecule is selected from the group consisting of DNA, RNA, protein, antibody, antigen, oligonucleotide and enzyme molecules.

8. Sensor array comprising at least two sensors according to any one of the preceding claims, wherein said sensors have a different recognition molecule and/or oligomer layer.

9. A method for manufacturing a sensor, said sensor having a substantially inert carrier substrate and at least one electrode positioned on at least part of said substrate, said method comprising the steps of :
- depositing an oligomer binding layer on said electrode, and
- binding a recognition molecule capable of recognition of an analyte in a fluid to said oligomer layer.

10. The method as recited in claim 9 wherein the recognition molecule is binded to the oligomer layer prior to the step of depositing the oligomer binding layer on said electrode.

11. The method as recited in claim 9 wherein the recognition molecule is binded to the oligomer layer after the step of depositing the oligomer binding layer on the electrode.

12. A method for locally depositing an oligomer binding layer on a sensor array comprising at least two sensors, the sensors having a substantially inert carrier substrate and at least one electrode positioned on at least part of said substrate, Said oligomer binding layer being deposited on at least one selected electrode of said array by applying a voltage higher than the threshold voltage for electrodeposition of said oligomer on said selected electrode while the voltage applied on at least part of the other electrodes of said array is lower than said threshold voltage.

13. Method for selectively depositing an oligomer layer on an electrode array structure, wherein said oligomer layer is deposited on a selected electrode of said electrode array by applying a voltage higher than the threshold voltage for electrodeposition of said oligomer over said selected electrode while the voltage applied over the other electrodes of said electrode array is lower than said threshold voltage.

14. The method as recited in claim 12 and 13 wherein the oligomer binding layer is a substituted backbone oligomer material.

15. The sensor as recited in claim 12 and 13 wherein the substituents on the backbone oligomer materials comprise at least one of the group of alkoxy-groups, hydroxyl-groups, amino-groups, epoxy-groups, thiol-groups and carboxyl-groups or any other group that is capable of binding molecules for detecting analytes in a fluid.

16. The sensor as recited in claim 12 and 13 wherein the oligomer layer is a substituted arylene alkenylene oligomer.

17. Sensor as in claims 12 and 13, wherein the oligomer layer is selected from the group consisting of 2,5-dimethoxy-1,4-bis(3,4,5-trimethoxystyryl)benzene, 2,5-alkoxy-1,4-bis(3,4,5-trimethoxystyryl)benzene and/or 1,4-bis(3,4,5-trimethoxystyryl)thiophene.

18. An oligomer material being a substituted bis(styryl)benzene oligomer materials or a substituted bis(styryl)thiophene oligomer materials.

19. The material as recited in claim 18 wherein the substituents are at least one of the group of alkoxy-groups, hydroxyl-groups, amino-groups, epoxy-groups, thiol-groups and carboxyl-groups or any other group that is capable of binding molecules for detecting analytes in a fluid.
